# EUROPEAN PATENT SPECIFICATION

(11) **EP 0 360 794 B1**
(45) Date of publication and mention of the grant of the patent: **03.08.1994**
(21) Application number: 88900494.1
(22) Date of filing: 16.10.1987
(51) Int. Cl.: C01B 11/02

(54) **METHOD OF PREPARING A MIXTURE OF CHLORINE CONTAINING SUBSTANCES INCLUDING CHLORINE DIOXIDE**
VERFAHREN ZUR HERSTELLUNG EINES GEMISCHES AUS CHLORAUFWEISENDEN VERBINDUNGEN EINSCHLIESSLICH CHLORDIOXYDEN
PROCEDE DE PREPARATION D'UN MELANGE DE SUBSTANCES CONTENANT DU CHLORE TEL QUE DU BIOXYDE DE CHLORE

(30) Priority: 15.05.1987 US 50922; 29.09.1987 US 102047
(43) Date of publication of application: 04.04.1990
(73) Proprietor: MASON, James A, Theodore, AL 36590 (US)
(72) Inventor: MASON, James A, Theodore, AL 36590 (US)
(74) Representative: Grünecker, Kinkeldey, Stockmair & Schwanhäusser Anwaltssozietät
(86) International application number: US8702686
(87) International publication number: WO8808823

(56) References cited:
- WO-A-85/04107
- CA-A- 959 238
- DE-A- 2 728 170
- FR-A- 2 345 393
- US-A- 4 084 747
- PATENT ABSTRACTS OF JAPAN, vol. 10, no. 208 (C-361)[2264], 22nd July 1986; & JP-A-61 048 404 (OSAKA SODA CO.) 10-03-1986, & CHEMICAL ABSTRACTS, vol.105, no. 8, page 151, abstract no. 63169u
- CHEMICAL ABSTRACTS, vol. 82, no. 22, 2nd June 1975, page 134, left-hand column, abstract no. 142249e, Columbus, Ohio, US; & CA-A-959 238 (CHEMICAL GENERATORS) 17-12-1974

## Description

This invention generally relates to a process for producing a mixture containing chlorine dioxide suitable for use as an oxidizing agent in various industrial processes. Exemplary industrial processes where chlorine dioxide may be used as an oxidizing agent or disinfectant include the disinfection of water and wastewater to destroy bacteria and/or pathogens, as a whitening agent in the paper industry and for water treatment in the oil recovery industry.

Other chlorine containing substances may be present in the mixture. It is believed that these other chlorine containing substances include chlorous acid, chloric acid and chlorine.

Methods and/or apparatuses for preparing chlorine containing substances including chlorine dioxide have been described in the prior art. US-A-4,250,144 describes a generating system for chlorine dioxide for use in the water or wastewater treatment industry. US-A-4,013,761 describes a system for generating chlorine dioxide including a generation vessel having leak inhibiting solvent weld joints with reducing couplings.

US-A-4,104,190 describes a system of generating chlorine dioxide from aqueous liquids containing alkali metal or alkaline earth metal chlorites, and compounds which liberate chlorine in water. US-A-4,534,952 describes a small scaled generator of chlorine dioxide for water treatment. US-A-4,504,442 describes the use of chlorine dioxide gas a a chemosterilizing agent particularly involving gas impermeable surfaces of implements commonly employed in the medical sciences. US-A-3,754,079 describes a process of preparing chlorine dioxide for use in the bleaching of wood pulp, fats, oils and flour. US-A-4,542,008 describes an electro-chemical process for producing chlorine dioxide from an aqueous solution of sodium chlorite. US-A-4,590,057 describes a process for the generation of chlorine dioxide from an aqueous solution of a metal chlorite and an oxidizing agent, preferably gaseous chlorine.

US-A-4,084,787 discloses the addition of an aqueous lactic acid solution to an aqueous solution of sodium chlorite. CA-A-959,238 discloses the introduction of quantities of chlorite and organic acid into a vessel separately in water soluble containers. Water is added to the vessel so that the water soluble containers dissolve and the reactants concomitantly intermix. WO85/04107 discloses mixing separate solutions just prior to use each individually containing chlorite and organic acid.

It is the abject of the present invention to provide a process for producing a mixture containing chlorine dioxide which may be transported in bulk and which exhibits little decomposition on storage. Said object is achieved by a process according to claims 1, 6 and 10.

According to the invention there is provided a method of forming chlorine dioxide and other chlorine containing compounds and/or substances in an aqueous solution from the reaction of lactic acid or citric acid with sodium chlorite to yield a salt of the acid and chlorous acid. This is the first reaction suspected in a series of reactions leading to the production of chlorine dioxide and is more particularly accomplished by introducing the lactic acid into water first and then mixing the contents of the reaction vessel by stirring. After the lactic acid and water are thoroughly mixed, the sodium chlorite is added to the mixture and again mixed by stirring.

This reaction is carried out at atmospheric pressure, at a pH less than 7 and at a temperature of less than 49°C (120° F), generally in the range of 16 to 27°C (60° F to 80° F), most preferably 17° C (62° F). The aqueous solution containing chlorine dioxide formed from the aforementioned reaction and subsequent reactions is stable and can be safely transported by common carrier, e.g., drums, tank truck or railway tank car, to the plant site for use.

It is believed that the aforementioned reaction is the first of a chain of reactions leading to the production of chlorine dioxide. These further reactions, generally, involve the oxidation of various organic or inorganic compounds of destruction of pathogens by either chlorous acid, chloric acid, chlorine dioxide or chlorine or a mixture of these substances and will be further described by use of chemical equations in the following section of this specification. The kinetics of these reactions are complex but it is believed that the reactions may occur more or less simultaneously with varying concentrations of the reaction and products being present as the reactions proceed to completion.

The chlorine containing compounds produced by the process of the present invention are intended for use as a bactericide to inhibit the growth of bacteria found in water and/or wastewater in order to make it potable or to disinfect it as the case may be. It is also intended to remove color, odor and taste problems that may be present in the water and/or wastewater. For example, such problems may be caused by algae, phenols and/or the presence of hydrogen sulfide. A particular advantage of the process of the present invention is that the production of trihalomethane, other undesirable polychlorinated hydrocarbons such as dioxine, chlorates and chlorites are minimized and/or eliminated. Other uses of the products obtained by the process of the present invention have been previously mentioned, e.g., as a bleaching agent in the paper industry and for water treatment in the oil recovery industry.

Note that it may be feasible to substitute other alkali metal or alkaline earth metal chlorites for sodium chlorite sometimes referred to in this specification. Also note, that it may be feasible to substitute other acids, such as citric, tartaric, malic and glycolic acids and/or other organic hydroxy acids and carboxylic acids for lactic acid.

One of the primary advantages of the process of the present invention is that it allows for the formation of a relatively stable mixture containing chlorine dioxide in an aqueous solution using bulk quantity reactants which allows for the production of the chlorine dioxide off site rather than on-site at a particular industrial site. Off site production is important because it allows for a much safer generating process for chlorine dioxide whereby the risk of fire and explosions are minimized. It is anticipated that the aqueous mixture containing chlorine dioxide would be shipped to the plant site by, e.g. tank truck or railway tank car. Furthermore, the process of the present invention allows for the mixing and formation of the chlorine dioxide in an aqueous solution involving bulk quantities and mixing ratios which are extremely simple and basic whereby more or less generally lesser-trained personnel can accomplish the production of the chlorine dioxide. This method allows for the delivery of a source of chlorine dioxide to a plant site by merely transporting an aqueous solution e.g., by a cool or a refrigerated means which is not now done because chlorine dioxide can not be safely transported and therefore is now generally generated on-site.

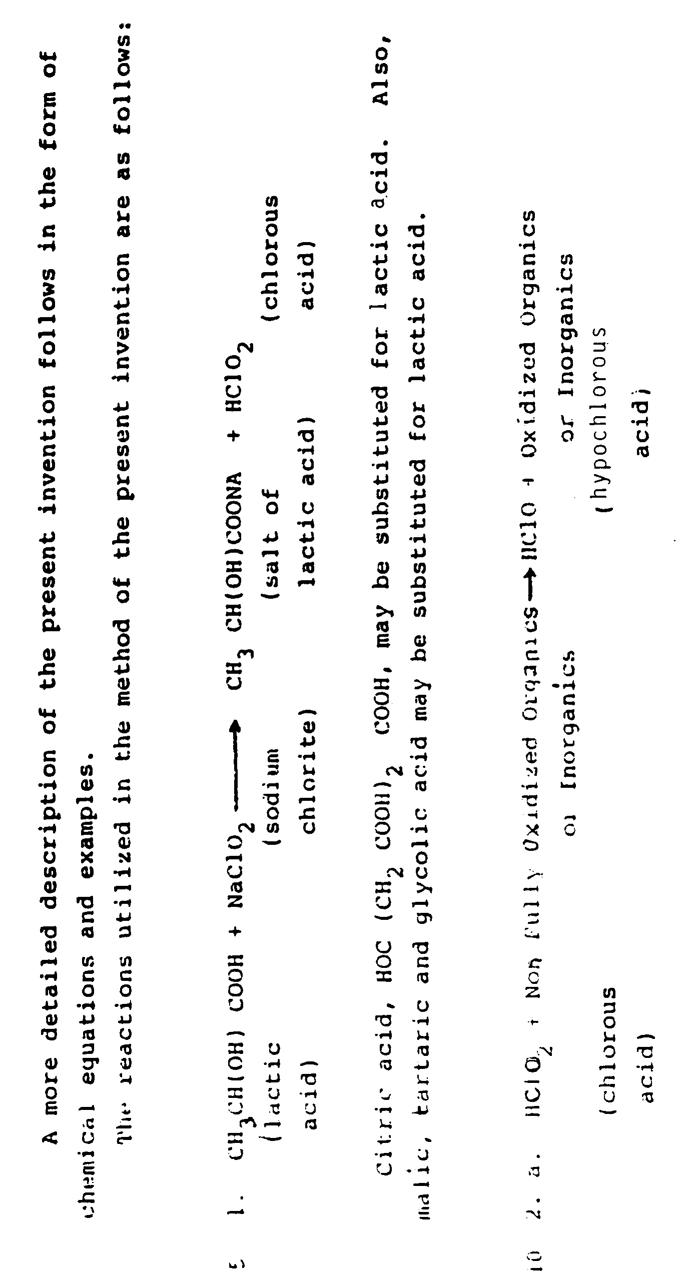

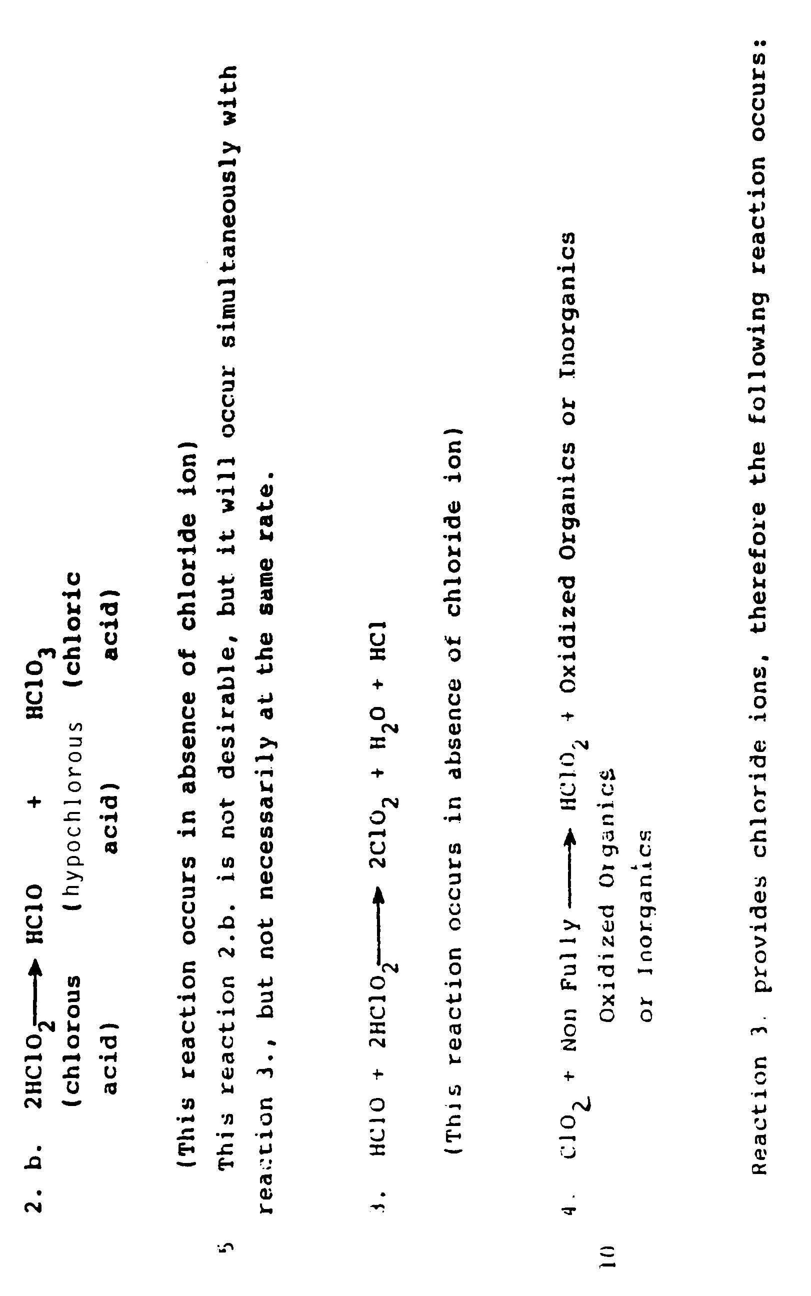

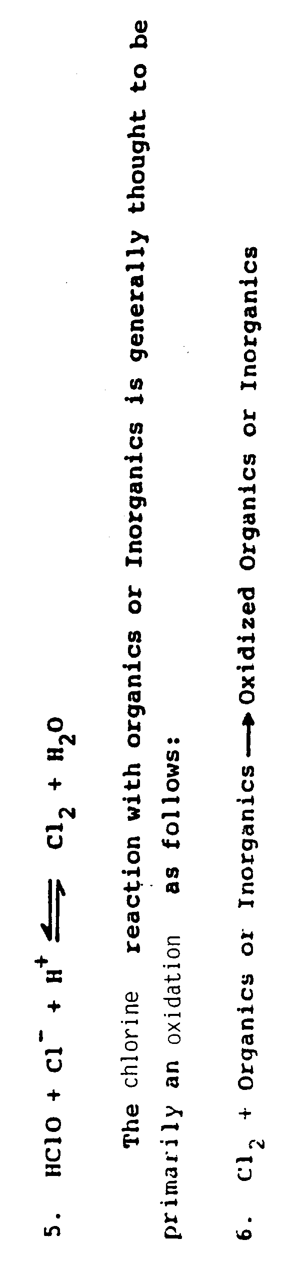

The above reactions are carried out at atmospheric pressure and at a pH less than 7. These reactions result in a relatively stable mixture of chlorine dioxide in an aqueous solution.

It is believed that the powerful oxidation and/or disinfection nature and characteristic of the process of the present invention is due to the action of chlorous acid, chloric acid, chlorine dioxide or chlorine, either jointly or singularly, or, a mixture of these chemical substances.

In practice, the reactants and reactions are produced by mixing bulk quantities of water, sodium chlorite and lactic acid to form an aqueous solution. In practice, about 1 part of lactic acid at a concentration of about 88% by volume, which is a food grade of lactic acid, is mixed with about 51 parts water and then mixed thoroughly by stirring. About three parts of sodium chlorite at a concentration of 25-26% by volume is then added to this solution and again mixed. In practice, the water could vary from 49-53 parts, the lactic acid could vary from 0.8-1.2 parts and the sodium chlorite could vary from 2.5-3.5 parts. Variations in the proportions or ratios of reactants in the ranges specified above results in variations in the concentrations of the end products of the reactions, e.g., the concentration of chlorine dioxide produced by these reactions. The 26% by volume of sodium chlorite and 88% by volume of lactic acid are commonly commercially available bulk quantities of these compounds and are generally provided to industry commercially in either drum lots or bulk quantities, for example, tank cars or tank trucks. Furthermore, note in the above reaction Number 1. that citric acid, HOC(CH₂COOH)₂COOH, at a concentration of about 55% by volume may be substituted for the lactic acid to produce a salt of citric acid and chlorous acid in an aqueous solution by using slightly different mixing ratios.

The above reactions are accomplished by mixing the reactants together at atmospheric pressure in an aqueous solution with the water temperature being less than 49°C (120°F), preferably in the range of 16 to 27°C (60° F to 80° F), most preferably 17°C (62° F). The higher water temperatures nearing 27° C (80° F) can be used if necessary to increase the reaction speed. Higher water temperatures may be feasible.

Explaining in more detail, small quantities may be obtained by the simple process of introducing into a small reaction vessel, (vessel should be one impervious to oxidants, such as chlorine or ClO₂) about 51 parts water, about 1 part 88% lactic acid food or technical grade, and about 3 parts 25% sodium chlorite. The lactic acid should be introduced into the water first, then mixed by stirring. After the water and lactic acid are thoroughly mixed add Sodium Chlorite and stir rapidly to obtain the final mixture. The water used should be at a temperature of not more than 49°C (120° F), preferably between 16 to 27°C (60°F - 80°F), and most preferably 17°C (62°F). The temperature of water will determine the reaction time required for the desired results. Higher water temperatures of course, result in more rapid formation of the end products. The final result being a mixture of HClO₂, HClO₃, and ClO₂ and Cl₂ in various concentrations,with the ClO₂ being at approximately 5000 ppm, in the range of 4000 ppm to 6000 ppm, using about 51 parts water, about one part lactic acid and about three parts sodium chlorite.

This mixture should immediately be transferred by pouring into a container impervious to the product, and capped tightly. It then may be stored at temperatures not to exceed 27° C (80° F) for long and possibly indefinite lengths of time. Lab tests have indicated that storage of up to 8 months is possible with little loss of initial strength.

If larger quantities are desire such as 208 l (55 gal), drum lots, similar steps are used. The drum should generally be a commercially available unit polyethylene lined, using about 193 l (51 gals) water, about 3,79l (1 gal) lactic acid and about 11,36 l (3 gals) sodium chlorite in the following order. Fill the drum to 1/2 to 2/3 capacity with water, i.e. about 106 - 144 l (28 - 38 gallons) add lactic acid, mixing thoroughly by stirring or agitation with, for example, a small commercially available agitator. Add the sodium chlorite and fill drum to capacity with additional water. Cap tightly immediately and store at below 27° C (80° F). About the same concentration of product i.e., about 5000 ppm, as explained above are obtained. The drum can then be shipped to the end use site but steps should be taken to insure that it is not exposed to temperatures in excess of 32° C (90° F) for extended times.

If tank car quantities are required, the same steps should be taken, except use a larger lined steel, stainless steel, polyethylene, or fiber glass tank equipped with an electrically powered agitator. If a 22712 l (600 gal) shipment is desired for example use the same ratio of about 51 parts water, about 1 part lactic acid, and about 3 parts Sodium Chlorite. Add approximately 2/3 of the total required water to the tank. Engage agitator pump, add lactic acid, allow to mix approximately 5 minutes, then leaving agitator engaged, add Sodium Chlorite and the balance of water to total about 51 parts required water. Transfer the mixture immediately to sealed commercial truck tanks suitable for transportation of this type product. Again, care must be taken to limit the maximum temperature for prolonged periods of time. Refrigerated units are commercially available to meet this requirement.

If the product is to be produced in very large quantities for immediate use such as in the bleaching of paper or flour, the warmer water temperatures may be necessary to obtain a more rapid reaction. Otherwise no particular emphasis should be placed on water temperature, and, tap water temperatures are acceptable within the limits herein described.

The ratio used in these examples result in a product strength of about 5000 ppm ClO₂. Stronger mixtures may be readily and safely obtained by changing these ratios of reactants. For example, to obtain a ClO₂ strength of approximately 15,000 ppm your ratio should be:
about 45 parts water
about 2½ parts lactic acid
about 7½ parts sodium chlorite
Higher strengths in excess of 100,000 ppm may be obtained. If this is desired more care should be taken to keep the final resulting product at below about 10°C (50° F) until it is introduced into the desired end use.

Please note that the mixing order of reactants must be carefully observed. If not, a violent reaction may result. It is believed that this is caused by the lactic acid being of different liquid consistency and/or density than the other constituents.

The aqueous solution resulting from the reactions above has a density of about 1.0039, a boiling point of about 101.6°C, a freezing point of about -3°C and a pH of approximately 4.7. The solution is completely miscible in water, has a pungent odor resembling chlorine and a color of clear to slightly amber.

As can be seen, the process of the present invention can be accomplished by easily mixing on a part to part basis commonly available commercial products in bulk quantities so as to produce the desired reactions. Granulated sodium chlorite can also be used to make up the bulk quantity of this aqueous solution.

The above reactions produce aqueous solutions containing very high concentrations of chlorine dioxide ranging from generally about 5000 ppm to about 80,000 ppm. Furthermore, the chlorine dioxide produced by the process of the present invention appears to have more oxidizing and pathogen destroying power on a per unit basis than chlorine dioxide produced by other methods. It is believed that this is due to the fact that the chlorine is available in the form of various substances including chlorous acid, chloric acid, chlorine dioxide and chlorine.

The invention will now be described by several examples which are given by way of illustration.

### EXAMPLE 1

The reaction was carried out using one 208 l (55 gallon) drum by volume of lactic acid at 88%, i.e., food grade, and three 208 l (55 gallon) drums of 26% sodium chlorite by volume to yield the desired reactions.

The lactic acid and sodium chlorite were first mixed in a large vessel of water having a temperature of approximately 17° C (62° F) being at atmospheric pressure.

### EXAMPLE 2

A granulated Sodium Chlorite was dissolved in water to form a 48% sodium chlorite solution according to standard published data on solubility of Sodium Chlorite. This solution was then combined with a solution of 88% lactic acid. An immediate reaction occurred forming a deep brown solution. This solution was tested and the presence of ClO₂ was detected. No attempt was made to ascertain the concentration of ClO₂ ppm in this solution.

### EXAMPLE 3

The same steps were taken as in Example 2 using 2 parts sodium chlorite, 1 part lactic acid, 4 parts water at approximately 16° C (60° F). Again the reaction showed the presence of ClO₂ after reaction in a closed vessel for approximately 30 minutes.

### EXAMPLE 4

The same steps were used as in Example 3 except the water was heated to a maximum temperature of approximately 49° C (120° F). The reaction appeared to take place much faster.

### EXAMPLE 5

A commercially available 26% solution of sodium chlorite was used with 88% lactic acid solution on a one to one basis The same reaction was observed as in Example 2.

### EXAMPLE 6

Same as Example 5, except 2 parts sodium chlorite 26% to 1 part lactic acid to 10 parts water at approximately 16° C (60° F). This formed a solution containing chlorine dioxide in excess of 80,000 ppm according to accepted tests.

### EXAMPLE 7

Same as Example 6, except 2-1/2 parts of sodium chlorite 26% was used to 1 part lactic acid to 10 parts water at approximately 16° C (60° F) with approximately the the same results as Example 6.

### EXAMPLE 8

Same as Example 7, except 3 parts sodium chlorite 26% to 1 part lactic acid 88% to 50 parts water at 16°C (60° F). This solution formed a solution containing 5000 plus or minus ppm ClO₂.

### EXAMPLE 9

A storage test was conducted where solutions were placed in 0,355l (12 oz.) amber bottles and capped. 1/3 were stored out of sunlight at approximately 22° C (72° F), 1/3 placed outside was exposed to sunlight and varying temperatures. 1/3 was placed in refrigerator at approximately 3° C (38° F).

Tests were conducted to determine loss of concentration and as expected the solution placed outdoors was the most unstable. The solution at 22° C (72° F) retained its concentration to within 2% plus or minus for at least 60 days at which time tests were discontinued.

The refrigerated solutions tested the same as the ones stored at 22° C (72° F) indoors, and were retained up to 120 days at which time sample tests were discontinued.

### EXAMPLE 10

Tests were conducted to determine if larger quantities could be commercially produced. 11,36 l (3 gals) of 26% sodium chlorite 3,79 l (1 gal) of 88% lactic acid, 193 l (51 gals) water in 208 l (55 gal) drums were combined using the following steps; (1) a 208 l (55 gal) drum was filled approximately 1/3 full with water and 3,79 l (1 gal) 88% lactic acid added and agitated to mix; (2) 11,36 l (3 gals) 26% sodium chlorite added and agitated; (3) drum was then filled with water and capped for 15 minutes; (4) drum uncapped and tested and found to contain ClO₂ at 5000 ppm plus or minus 2%.

### EXAMPLE 11

Same as Example 10 except 22,7 l (6 gal) sodium chlorite, 7,56 l (2 gals) lactic acid and 178 l (47 gals) water were combined to produce a solution containing 10,000 + ppm ClO₂.

### EXAMPLE 12

Same as Example 10 except 30,3 l (8 gals) sodium chloride, 9,46 l (2½ gals) lactic acid, 168 l (44½ gal) water were combined. Test showed 18.000 plus or minus 2% ppm ClO₂.

It was found that a solution of 10,000 ppm may be stored in drums without loss of appreciable concentrations up to 90 days.

### EXAMPLE 13

Tests were conducted in a Northwest Florida potable surface water treatment facility. The facility treats an average of 60,6 million liter per day (16 MGD (million gallons per day)). A 208 l (55 gal) drum of approximate 5000 ppm ClO₂ solution was used and fed into the system prior to flocculation, at a rate of 3,79 l (1 gal) solution per 3,79 million l (million gal) water.

Samples were taken and tested on site and also by a certified water testing laboratory in N.W. Florida.

The results showed 0 total coliforms, trihalomethane production was below detection level, and no chlorites or chlorates were detectable in the samples taken.

It is believed that the most desirable concentration for the treatment of potable water is about 5000 ppm ClO₂ due to ease of handling and effectiveness of product as a disinfectant.

### EXAMPLE 14

Grab samples were taken from a large Southwestern city potable (surface water) water filtration system. The samples were treated by applying 5000 ppm ClO₂ solution to the water in the following part to part ratios: 1,89 l (1/2 gal) solution per 3,79 million l (million gals) water; 3,79 l (1 gal) solution per 3,79 million l (million gals) water and 7,57 l (2 gal) solution per 3,79 million (million gals) water. The results were the same as in Example 13 when mixing 7,57 l (2 gal) solution per 3,79 million 1 (million gals) water.

### EXAMPLE 15

Grab samples were taken from a South Alabama municipal potable (surface water) and treated the same as in Example 13, with the same results as Example 13.

### EXAMPLE 16

Tests were conducted in a small South Louisiana city. Groundwater is pumped from a depth in excess of 457,2 m (1500 feet). 5 wells supply the city's total water requirements. High concentrations of H₂S as evidenced by the strong sulfur taste and rotten egg smell. The H₂S levels tested to show 5 ppm. A 208 l (55 gal) drum of 5000 ppm ClO₂ solution was delivered to each well location and applied to the raw water at each wellhead at a rate of 1 part solution per 3,79 million l per day (M.G.D). Samples of water at each location were taken periodically over a period of 30 days. Results showed complete elimination of H₂S taste and odor. Tests by a certified laboratory in South Louisiana showed no adverse effects in final treated water.

## Claims

1. A process for producing a mixture containing chlorine dioxide comprising the steps of:
(a) forming a first solution by introducing lactic acid into water in a reaction vessel;
(b) mixing said first solution by stirring;
(c) thereafter forming a second solution by introducing sodium chlorite into said first solution following said mixing; and
(d) mixing said second solution by stirring.

2. The process of claim 1, wherein a total of 49 to 53 parts water by volume, 0.8 to 1.2 parts lactic acid by volume and 2.5 to 3.5 parts sodium chlorite by volume are used.

3. The process of claim 1 or 2 wherein the water temperature ranges from 16 to 27°C (60 to 80°F).

4. The process of claim 1 or 2, wherein chlorine dioxide is formed having a concentration varying from 4000 to 6000 ppm.

5. The process of claim 1, wherein the chlorine dioxide product is stable and maintains its original concentration within 2% of its original value for at least 30 days when stored at temperatures not exceeding 27°C (80°F).

6. A process for producing a mixture containing chlorine dioxide comprising the steps of:
(a) forming a first solution by introducing an organic hydroxy acid or carboxylic acid into water in a reaction vessel;
(b) mixing said first solution by stirring;
(c) forming a second solution by introducing an alkali metal chlorite or alkaline earth metal chlorite into said first solution following said mixing; and
(d) mixing said second solution by stirring.

7. The process of claim 6, wherein the water temperature ranges from 16 to 27°C (60° to 80° F).

8. The process of claim 6, wherein chlorine dioxide is formed having a concentration varying from 4,000 to 6,000 ppm.

9. The process of claim 6, wherein the chlorine dioxide product is stable and maintains its original concentration within 2% of its original value for at least 30 days when stored at temperatures not exceeding 27°C (80°F).

10. A process for producing a mixture containing chlorine dioxide comprising the steps of:
(a) forming a first solution by introducing lactic acid into water in a reaction vessel;
(b) mixing said first solution by stirring;
(c) thereafter forming a second solution by introducing sodium chlorite into said first solution following said mixing;
(d) mixing said second solution by stirring; and
(e) storing said stirred second solution containing chlorine dioxide at temperatures not exceeding 27°C (80° F), wherein the chlorine dioxide is stable and maintains its original concentration within 2% of its original value for at least 30 days.

11. A modified process as claimed in claim 1 or 10 wherein citric acid is substituted for lactic acid.

12. A modified process as claimed in claim 1 or 10, wherein malic acid is substituted for lactic acid.

13. A modified process as claimed in claim 1 or 10 wherein tartaric acid is substituted for lactic acid.

14. A modified process as claimed in claim 1 or 10, wherein glycolic acid is substituted for lactic acid.

15. The process of claim 10, wherein a total of 49 to 53 parts water by volume, 0.8 to 1.2 parts lactic acid by volume and 2.5 to 3.5 parts sodium chlorite by volume are used.

16. The process of claim 15, wherein chlorine dioxide is formed having a concentration varying from 4,000 to 6,000 ppm.

## Patentansprüche

1. Verfahren zum Herstellen eines Gemisches, welches Chlordioxid enthält, umfassend die Schritte:
(a) Bilden einer ersten Lösung durch Einbringen von Milchsäure in Wasser in einem Reaktionsgefäß;
(b) Vermischen der ersten Lösung durch Rühren;
(c) danach Bilden einer zweiten Lösung durch Einbringen von Natriumchlorit in die erste Lösung im Anschluß an das Vermischen; und
(d) Vermischen der zweiten Lösung durch Rühren.

2. Verfahren nach Anspruch 1, worin insgesamt 49 bis 53 Volumenteile Wasser, 0,8 bis 1,2 Volumenteile Milchsäure und 2,5 bis 3,5 Volumenteile Natriumchlorit verwendet werden.

3. Verfahren nach Anspruch 1 oder 2, worin die Wassertemperatur im Bereich von 16 bis 27°C (60 bis 80°F) liegt.

4. Verfahren nach Anspruch 1 oder 2, worin Chlordioxid mit einer Konzentration, die zwischen 4000 und 6000 ppm schwankt, gebildet wird.

5. Verfahren nach Anspruch 1, worin das Chlordioxidprodukt stabil ist und seine ursprüngliche Konzentration innerhalb von 2% ihres ursprünglichen Werts mindestens 30 Tage lang beibehält, wenn es bei Temperaturen gelagert wird, die 27°C (80°F) nicht überschreiten.

6. Verfahren zum Herstellen eines Gemisches, das Chlordioxid enthält, umfassend die Schritte:
(a) Bilden einer ersten Lösung durch Einbringen einer organischen Hydroxycarbonsäure oder Carbonsäure in Wasser in einem Reaktionsgefäß;
(b) Vermischen der ersten Lösung durch Rühren;
(c) Bilden einer zweiten Lösung durch Einbringen eines Alkalimetallchlorits oder Erdalkalimetallchlorits in die erste Lösung im Anschluß an das Vermischen; und
(d) Vermischen der zweiten Lösung durch Rühren.

7. Verfahren nach Anspruch 6, worin die Wassertemperatur im Bereich von 16 bis 27°C (60 bis 80°F) liegt.

8. Verfahren nach Anspruch 6, worin Chlordioxid mit einer Konzentration, die von 4000 bis 6000 ppm schwankt, gebildet wird.

9. Verfahren nach Anspruch 6, worin das Chlordioxidprodukt stabil ist und seine ursprüngliche Konzentration innerhalb von 2% ihres ursprünglichen Werts mindestens 30 Tage lang beibehält, wenn es bei Temperaturen gelagert wird, die 27°C (80°F) nicht überschreiten.

10. Verfahren zum Herstellen eines Gemisches, das Chlordioxid enthält, umfassend die Schritte:
(a) Bilden einer ersten Lösung durch Einbringen von Milchsäure in Wasser in einem Reaktionsgefäß;
(b) Vermischen der ersten Lösung durch Rühren;
(c) danach Bilden einer zweiten Lösung durch Einbringen von Natriumchlorit in die erste Lösung im Anschluß an das Vermischen;
(d) Vermischen der zweiten Lösung durch Rühren; und
(e) Lagern der gerührten zweiten Lösung, die Chlordioxid enthält, bei Temperaturen, die 27°C (80°F) nicht überschreiten, worin das Chlordioxid stabil ist und seine urspüngliche Konzentration innerhalb von 2% ihres ursprünglichen Werts mindestens 30 Tage lang beibehält.

11. Modifiziertes Verfahren nach Anspruch 1 oder 10, worin Milchsäure durch Citronensäure ersetzt wird.

12. Modifiziertes Verfahren nach Anspruch 1 oder 10, worin Milchsäure durch Äpfelsäure ersetzt wird.

13. Modifiziertes Verfahren nach Anspruch 1 oder 10, worin Milchsäure durch Weinsäure ersetzt wird.

14. Modifiziertes Verfahren nach Anspruch 1 oder 10, worin Milchsäure durch Glycolsäure ersetzt wird.

15. Verfahren nach Anspruch 10, worin insgesamt 49 bis 53 Volumenteile Wasser, 0,8 bis 1,2 Volumenteile Milchsäure und 2,5 bis 3,5 Volumenteile Natriumchlorit verwendet werden.

16. Verfahren nach Anspruch 15, worin Chlordioxid mit einer Konzentration, die zwischen 4000 und 6000 ppm schwankt, gebildet wird.

## Revendications

1. Procédé de production d'un mélange contenant du bioxyde de chlore, comprenant les étapes de :
(a) la formation d'une première solution par l'introduction d'acide lactique dans de l'eau dans un réacteur;
(b) le mélange de ladite première solution par agitation;
(c) la formation ultérieure d'une deuxième solution par l'introduction de chlorite de sodium dans ladite première solution à la suite dudit mélange; et
(d) le mélange de ladite deuxième solution par agitation.

2. Procédé selon la revendication 1, dans lequel un total de 49 à 53 parties d'eau en volume, de 0,8 à 1,2 parties d'acide lactique en volume et de 2,5 à 3,5 parties de chlorite de sodium en volume sont utilisées.

3. Procédé selon la revendication 1 ou 2, dans lequel la température de l'eau est comprise entre 16 et 27°C (60 et 80°F).

4. Procédé selon la revendication 1 ou 2, dans lequel du bioxyde de chlore est formé avec une concentration variant entre 4000 et 6000 ppm.

5. Procédé selon la revendication 1, dans lequel le produit de bioxyde de chlore est stable et conserve sa concentration d'origine à moins de 2% de sa valeur d'origine pendant au moins 30 jours lorsqu'il est stocké à des températures n'excédant pas 27°C (80°F).

6. Procédé de production d'un mélange contenant du bioxyde de chlore, comprenant les étapes de :
(a) la formation d'une première solution par l'introduction d'un acide hydroxylique organique ou d'un acide carboxylique organique dans de l'eau dans un réacteur;
(b) le mélange de ladite première solution par agitation;
(c) la formation d'une deuxième solution par l'introduction de chlorite de métal alcalin ou de chlorite de métal alcalino-terreux dans ladite première solution à la suite dudit mélange; et
(d) le mélange de ladite deuxième solution par agitation.

7. Procédé selon la revendication 6, dans lequel la température de l'eau est comprise entre 16 et 27°C (60 et 80°F).

8. Procédé selon la revendication 6, dans lequel du bioxyde de chlore est formé avec une concentration variant entre 4000 et 6000 ppm.

9. Procédé selon la revendication 6, dans lequel le produit de bioxyde de chlore est stable et conserve sa concentration d'origine à moins de 2% de sa valeur d'origine pendant au moins 30 jours lorsqu'il est stocké à des températures n'excédant pas 27°C (80°F).

10. Procédé de production d'un mélange contenant du bioxyde de chlore, comprenant les étapes de :
(a) la formation d'une première solution par l'introduction d'acide lactique dans de l'eau dans un réacteur;
(b) le mélange de ladite première solution par agitation;
(c) la formation ultérieure d'une deuxième solution par l'introduction de chlorite de sodium dans ladite première solution à la suite dudit mélange;
(d) le mélange de ladite deuxième solution par agitation; et
(e) le stockage de ladite deuxième solution contenant du bioxyde de chlore à des températures n'excédant pas 27°C (80°F), dans lequel le bioxyde de chlore est stable et conserve sa concentration d'origine à moins de 2% de sa valeur d'origine pendant au moins 30 jours.

11. Procédé modifié comme revendiqué dans la revendication 1 ou 10, dans lequel de l'acide citrique est substitué à l'acide lactique.

12. Procédé modifié comme revendiqué dans la revendication 1 ou 10, dans lequel de l'acide malique est substitué à l'acide lactique.

13. Procédé modifié comme revendiqué dans la revendication 1 ou 10, dans lequel de l'acide tartrique est substitué à l'acide lactique.

14. Procédé modifié comme revendiqué dans la revendication 1 ou 10, dans lequel de l'acide glycolique est substitué à l'acide lactique.

15. Procédé selon la revendication 10, dans lequel un total de 49 à 53 parties d'eau en volume, de 0,8 à 1,2 parties d'acide lactique en volume et de 2,5 à 3,5 parties de chlorite de sodium en volume sont utilisées.

16. Procédé selon la revendication 15, dans lequel du bioxyde de chlore est formé avec une concentration variant entre 4000 et 6000 ppm.
